# EUROPEAN PATENT SPECIFICATION

(11) **EP 3 857 225 B1**
(45) Date of publication and mention of the grant of the patent: **16.04.2025**
(21) Application number: 19866446.8
(22) Date of filing: 27.09.2019
(51) Int. Cl.: G01N 33/543

(54) **COMPLEX STABILIZING COMPONENTS AND DETECTION METHODS THEREOF**
KOMPLEXE STABILISIERENDE KOMPONENTEN UND NACHWEISVERFAHREN DAFÜR
COMPOSANTS DE STABILISATION COMPLEXES ET LEURS PROCÉDÉS DE DÉTECTION

(30) Priority: 28.09.2018 US 201862739132 P
(43) Date of publication of application: 04.08.2021
(73) Proprietor: Siemens Healthcare Diagnostics Inc., Tarrytown, NY 10591 (US)
(72) Inventor: VITZTHUM, Frank, New City, New York 10956 (US); D'AGOSTINO, Paul, Middletown, New York 10941 (US); CORTESE, Kayla, Mahwah, New Jersey 07430 (US)
(74) Representative: Schweitzer, Klaus
(86) International application number: PCT/US2019/053385
(87) International publication number: WO 2020/069271

(56) References cited:
- US-A1- 2015 232 541
- US-B1- 6 242 195
- MUKHERJEE SOMNATH ET AL: "Engineered synthetic antibodies as probes to quantify the energetic contributions of ligand binding to conformational changes in proteins", JOURNAL OF BIOLOGICAL CHEMISTRY, 10 January 2018 (2018-01-10), pages 2815 - 2828, XP055853500, Retrieved from the Internet <URL:https://www.jbc.org/action/showPdf?pii=S0021-9258(17)47588-0> [retrieved on 20211021], DOI: 10.1074/jbc.RA117.000656
- EASTERBROOK-SMITH S B ET AL: "The role of Fc:Fc interactions in insoluble immune complex formation and complement activation", MOLECULAR IMMUNOLOGY, PERGAMON, GB, vol. 25, no. 12, 1 December 1988 (1988-12-01), pages 1331 - 1337, XP023681530, ISSN: 0161-5890, [retrieved on 19881201], DOI: 10.1016/0161-5890(88)90048-X
- S. A. KAZANE ET AL: "Site-specific DNA-antibody conjugates for specific and sensitive immuno-PCR", PROCEEDINGS OF THE NATIONAL ACADEMY OF SCIENCES, vol. 109, no. 10, 6 March 2012 (2012-03-06), pages 3731 - 3736, XP055076255, ISSN: 0027-8424, DOI: 10.1073/pnas.1120682109
- SARIT S. AGASTI ET AL: "Photocleavable DNA Barcode-Antibody Conjugates Allow Sensitive and Multiplexed Protein Analysis in Single Cells", JOURNAL OF THE AMERICAN CHEMICAL SOCIETY, vol. 134, no. 45, 2 November 2012 (2012-11-02), pages 18499 - 18502, XP055629087, ISSN: 0002-7863, DOI: 10.1021/ja307689w

## Description

### FIELD OF THE DISCLOSURE

This disclosure is directed to the field of immunoassay. Specifically, the disclosure provides complexes, methods, and kits for accurate and sensitive detection and measurement of targets or components potentially in a sample or medium, where the targets are typically associated with conditions or disease states.

### BACKGROUND

Since the advent of diagnostic assays for detecting disease biomarkers, there has been continuous drive for innovative changes to improve these diagnostic tools. There is an ongoing need to obtain faster and effective detection methods, particularly in this day and age of global air travel and the spread of infectious disease. While detection of disease biomarkers may be applicable for testing for an infectious outbreak, diagnosing and monitoring disease in a patient, such as those, for example, related to cardiology, autoimmunity, oncology, infection, etc. Challenges arise in immunoassays with respect to sample size, immobilization and/or capture, interference between antibodies and proteins, which affect the accuracy, sensitivity, and specificity, and the assays can be laborious as well as expensive.

The article of Somnath Mukherjee ET AL: "Engineered synthetic antibodies as probes to quantify the energetic contributions of ligand binding to conformational changes in proteins", in: J. Biol. Chem. 293 [2018] 2815 - 2828, investigates energetic contributions of distinct conformational dynamics to specific biological outputs. A unique approach exploiting conformation-specific and regio-specific synthetic antibodies (sABs) are used to probe the energetic contributions of ligand binding to conformation changes. It is determined that the binding of a closed conformation-specific sAB (sAB-11M) to MBP in the absence of maltose is entropically driven, providing new insight into designing antibody-stabilized protein interactions.

The article of S.B. Easterbrook-Smith, "The role of Fc : Fc interactions in insoluble immune complex formation and complement activation ", in: Molecular Immunology 25 [1988] 1331 - 1337 is directed to the effects of Fc-specific complement protein on the initial rate of formation of complexes comprising ovalbumin and anti-ovalbumin IgG. It describes importance of interaction between the Fc portion of IgG molecules in the formation of insoluble immune complex.

Precision medicine requires *in vitro* diagnostic (IVD) assays with high diagnostic accuracy. A means of achieving higher diagnostic accuracy is through IVD assays with higher analytical accuracy. The analytical sensitivity and the specificity with which the measured is determined are key aspects that contribute to the analytical accuracy of an assay. For immunoassays, the specific binding between antibodies and antigens is leveraged to provide an accurate detection or measurement. Though antibodies and antigens typically bind with high affinity and specificity, further improvement is needed to allow for even more accurate assays.

There is an increasing clinical need for high sensitivity detection of human immunoglobulins associated with disease states (e.g. infectious or autoimmune diseases). Thus, state of the art IVD immunoassays must employ novel biochemical techniques to improve the fixed detection capabilities associated with the CENTAUR and ATELLICA IA Analyzer platforms.

It is therefore an object of this disclosure to provide an *in vitro* diagnostic assay with improved sensitivity and accuracy for detection and measurement of targets of interest associated with disease. There is an ongoing need for a highly sensitive detection system of, for example, human immunoglobulins associated with disease states (e.g., infection or autoimmune diseases). Moreover, innovative assays are needed to improve the fixed detection capabilities associated with the CENTAUR and ATELLICA IA ANALYZER platforms.

### SUMMARY

In accordance with the foregoing objectives and others, the present disclosure provides in one aspect, a method for determining the presence or relative amounts of at least one target or component in a sample or medium.

One aspect of the invention relates to an immune complex, comprising:
(a) a target conjugate pair, where the target conjugate pair comprises a target (e.g., antibodies, including autoantibodies, such as thyroid peroxidase autoantibodies (aTPO)) and a conjugate associated with the target; and
(b) a stabilizing component,
   where the stabilizing component binds to the target conjugate pair, where the association constant (Ka) of the complex is greater than the Ka of the complex without the stabilizing component, as defined by the claims.

One aspect of the present invention relates to a method, comprising: contacting a sample being tested for the presence of a target and a mixture of a conjugate associated with the target and a stabilizing component, where the target and the conjugate associated with the target form a target conjugate pair, where the stabilizing component is linked to the target conjugate pair to form a complex as defined in claims 1-7, where the association constant (Ka) of the complex is greater than the Ka of the complex without the stabilizing component.

The present invention further relates to a kit, comprising: a conjugate associated with a target and a stabilizing component configured to form the complex as defined by the claims.

### BRIEF DESCRIPTION OF FIGURES

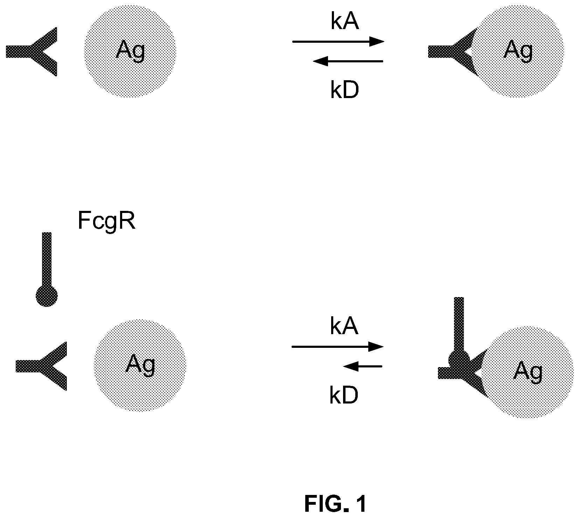

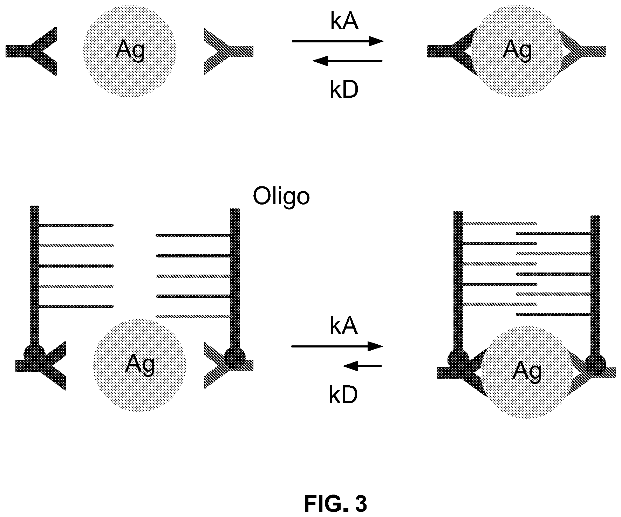

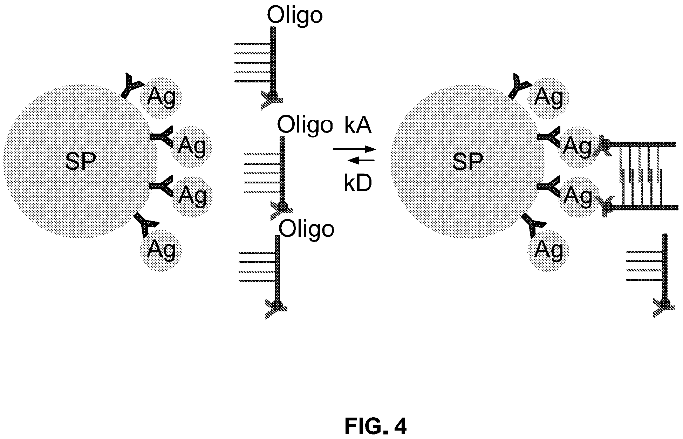

FIG. 1 demonstrates binding of a stabilizing component to form a complex comprising a target (e.g., antigen (Ag)), a target-binding entity (e.g., antibody that recognizes the Ag; depicted by "Y"), and a stabilizing component (e.g., Fc gamma receptor; FcyR) bound to the target-binding entity. The affinity of binding the immune complex with the stabilizing component (bottom reaction) is higher than the affinity of the complex without the stabilizing component (top reaction). This is because the dissociation constant (Kd) of the complex with the stabilizing component (bottom reaction) is less than or reduced compared to the Kd of the complex without the stabilizing components (top reaction).

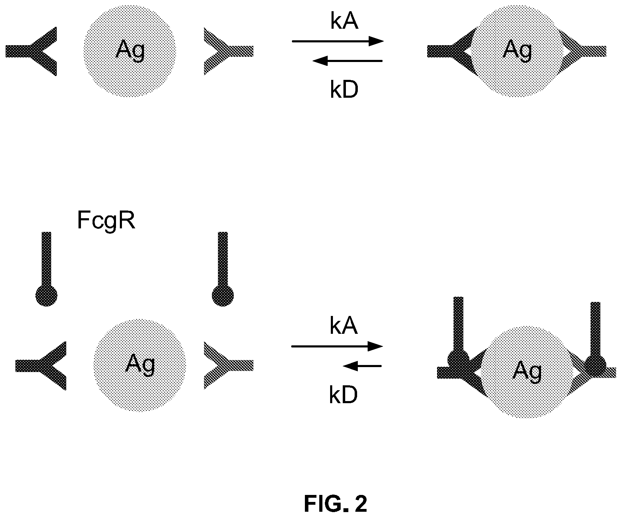

FIG. 2 demonstrates binding of two stabilizing components to form a complex comprising a target (e.g., antigen (Ag)), two target-binding entities (e.g., antibody that recognizes the Ag target; depicted by "Y"), and two stabilizing components (e.g., Fc gamma receptor; FcyR) bound to each target-binding entity in the architecture of a typical sandwich immunoassay. The affinity for the stabilizing components to bind (bottom reaction) is higher than the affinity of the complex without the stabilizing components (top reaction). This is because the dissociation constant (Kd) of the complex (bottom reaction) is less than or reduced compared to the Kd of the complex without the stabilizing components (top reaction).
(not claimed) FIG. 3 demonstrates binding of two stabilizing components to a target (e.g., antigen (Ag)) to form a complex, where each stabilizing component has a target-binding entity (e.g., antibody that recognizes the Ag target; depicted by "Y") bound to an oligonucleotide ("oligo"), where each oligonucleotide has a nucleic acid sequence, and the nucleic acid sequences are complementary and hybridize. This is exemplary of complementary single-stranded DNA (ssDNA) oligonucleotides that are each, separately conjugated to an anti-human IgG (with affinity to an antigen, Ag) and used as stabilizing components. When an anti-human IgG is conjugated to a ssDNA oligonucleotide, the dissociation constant (Kd) of the complex (bottom reaction) is lowered and the affinity is increased as compared to the Kd and affinity of the complex formed without the ssDNA oligonucleotides (top reaction).
(not claimed) FIG. 4 demonstrates binding of stabilizing components to a target conjugate pair, where the target conjugate pair comprises a target (e.g., Ag) and the conjugate associated with the target, where the conjugate comprises a target-binding entity (e.g., antibody that recognizes the Ag target; depicted by "Y") bound to a support (e.g., solid phase "SP"). The stabilizing component has a target-binding entity (e.g., antibody that recognizes the Ag target; depicted by "Y") bound to an oligonucleotide ("oligo"), where each oligonucleotide has a nucleic acid sequence, where the nucleic acid sequences are complementary and hybridize. This is exemplary of ssDNA oligonucleotides used as stabilizing components in an immunoassay format that utilizes an antibody conjugated to a SP support to capture an antigen, Ag. Secondary antibodies conjugated to complementary ssDNA oligonucleotides may hybridize with one another to stabilize the complex and increase overall avidity. The dissociation constant (Kd) of the complex (on the right) is less than the Kd of the complex without the stabilizing components (on the left).

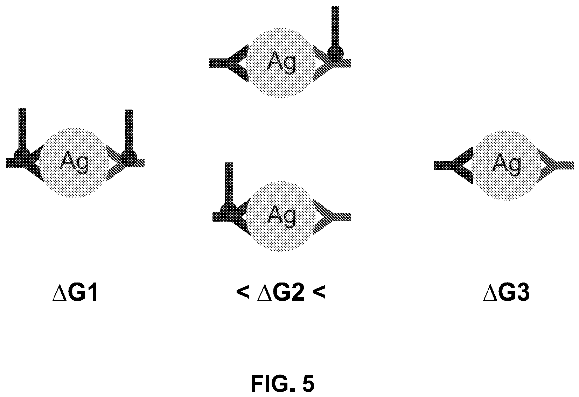

FIG. 5 demonstrates the decreasing free enthalpy also known as free energy (G) in the presence of stabilizing components. A stable complex comprising a target (e.g., Ag), a target-binding entity (e.g., antibody that recognizes the Ag target; depicted by "Y"), and a stabilizing component (e.g., Fc gamma receptor; FcyR), where the most stable complex comprises two stabilizing components has the lowest free energy (left complex), a less stable complex comprises one stabilizing component (middle complexes), and the least stable complex comprises no stabilizing components (right complex). The free energy is reduced through the lowering of free energy and/or through an entropy driven process due to the additional binding event(s) of the stabilizing component to the complex. The difference of the free energy (delta G, ΔG) reduction is higher when the stabilizing component binds. Free energy should be further reduced as more stabilizing components contribute to the binding. The dissociation constant (Kd) decreases from the right complex, to the middle complex to the left complex; and the association constant (Ka) decreases from the left complex comprising two stabilizing components to the middle complex to the right complex with none stabilizing components. A reduction in free energy will lead to a more stable complex and improved detection.

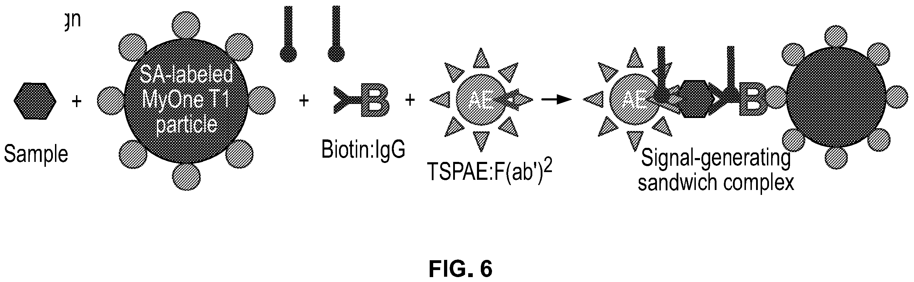

FIG. 6 demonstrates an exemplary modified acridinium ester (AE) sandwich immunoassay, where the modification is directed to the use of two stabilizing components (e.g., FcyR). The complex comprises two target conjugate pairs, where a first target conjugate pair comprises a target from a sample (depicted by a hexagon) bound to a conjugate comprising an antibody fragment (e.g., F(ab')₂) bound to a detectable label (e.g., acridinium ester; depicted by a sun symbol and "AE"), where the antibody fragment binds the target, and a second target conjugate pair comprising the same target from the sample bound to a conjugate comprising an antibody (e.g., IgG) bound to a support (e.g., paramagnetic bead), where the antibody and support are linked by a binding pair (e.g., biotin/streptavidin), where the streptavidin is pre-coated onto the support and the biotin is conjugated to the antibody that binds the target, where two stabilizing components (e.g., FcyR) are linked to the antibody fragment of the first target conjugate pair and the antibody of the second conjugate pair.

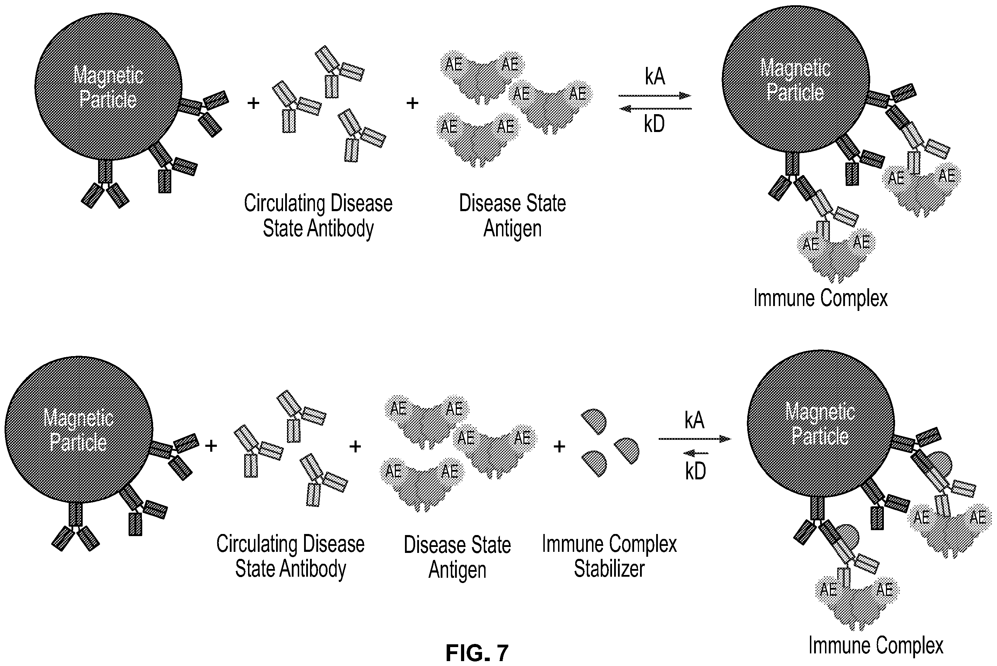

FIG. 7 depicts a modified acridinium ester (AE) immunoassay to detect antibodies (e.g., circulating disease state antibodies), where the modification is directed to the use of two stabilizing components. The complex comprises two target conjugate pairs, where a first target conjugate pair comprises a target from a sample (depicted by a circulating disease state antibody) bound to a conjugate comprising a disease state antigen (that recognizes the target) bound to a detectable label (e.g., acridinium ester; depicted by "AE"), and a second target conjugate pair comprising the same target from the sample bound to a conjugate comprising an antibody (e.g., IgG) that recognizes the target bound to a support (e.g., paramagnetic bead), where two stabilizing components (e.g., FcyR; depicted by half circles) are linked to the target conjugate pair.

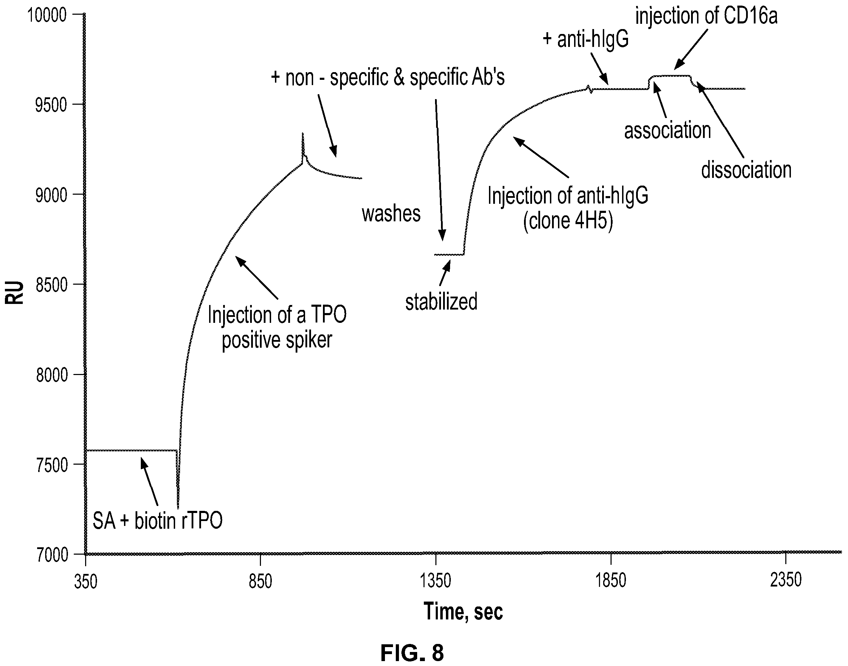

FIG. 8 illustrates a sensorgram of a thyroid peroxidase autoantibody (aTPO) positive spiker injected on to a BIACORE chip pre-loaded with streptavidin (SA) and biotin conjugated to recombinant TPO (rTPO), followed by removal of non-specific bindings by extensive washing. After which, anti-human immunoglobulin G monoclonal antibody (anti-hIgG mAb) (clone 4H5) and CD16a are sequentially injected (depicted by the upper left and right curves, respectively).

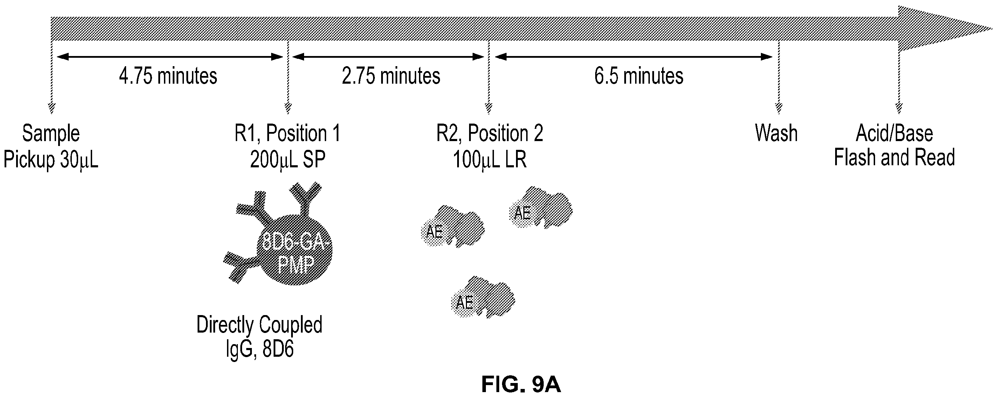

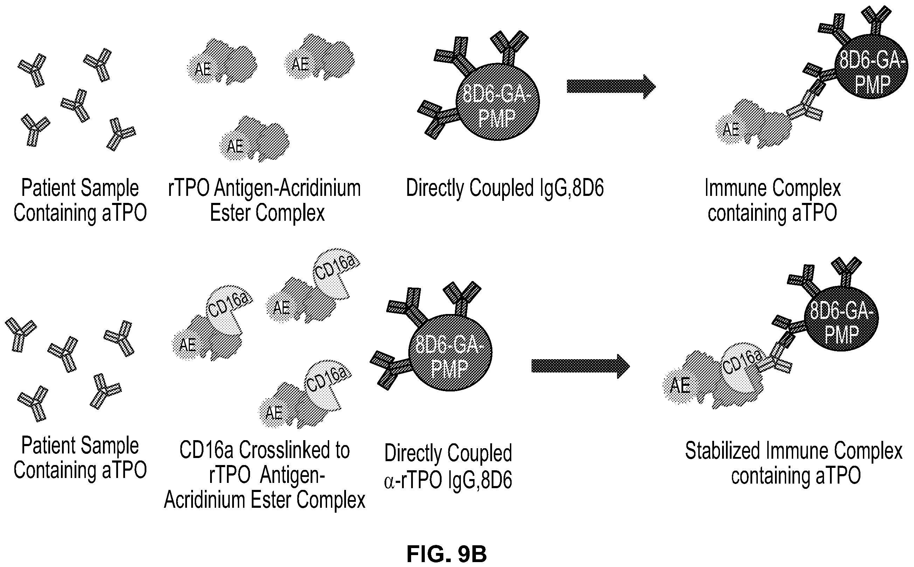

FIG. 9A outlines an exemplary capture assay protocol; and FIG. 9B provides an illustration.

### DETAILED DESCRIPTION

Detailed embodiments of the present disclosure are disclosed herein; however, it is to be understood that the disclosed embodiments are merely illustrative of the disclosure that may be embodied in various forms. In addition, each of the examples given in connection with the various embodiments of the disclosure is intended to be illustrative, and not restrictive.

All terms used herein are intended to have their ordinary meaning in the art unless otherwise provided. All concentrations are in terms of percentage by weight of the specified component relative to the entire weight of the topical composition, unless otherwise defined.

As used herein, "a" or "an" shall mean one or more. As used herein when used in conjunction with the word "comprising," the words "a" or "an" mean one or more than one. As used herein "another" means at least a second or more.

By "consist essentially" it is meant that the ingredients include only the listed components along with the normal impurities present in commercial materials and with any other additives present at levels which do not affect the operation of the embodiments disclosed herein, for instance at levels less than 5% by weight or less than 1% or even 0.5% by weight.

The disclosure is generally directed to methods of detecting the presence of a target in a sample where there is improved detection and accuracy in view of a heightened sensitivity achieved by complexes comprising a stabilizing component. This increased sensitivity may be achieved by providing a complex having a stabilizing component, where the association constant (Ka) of the complex is greater than the Ka of the complex without the stabilizing component, the Ka of the complex without the stabilizing component is less than the Ka of the complex with the stabilizing component, the dissociation constant (Kd) of the complex is less than the Kd of a complex without the stabilizing component, and the Kd of the complex without the stabilizing component is greater than the Kd of the complex.

Introducing a biochemical moiety as a stabilizing component has the potential to improve assay robustness while also increasing low end sensitivity. There are several moieties that can be used to improve stability of immune complexes in the assays or methods of the disclosure, which can be leveraged to increase performance of respective assays. In the invention, when a binding event occurs in the presence of the stabilizing component gamma receptor aka CD16a, the formation of a complex which undergoes a conformational change in the presence of the stabilizing component may contributes to a more stable complex. Additionally but not part of the present invention, complementary single stranded DNA (ssDNA) oligonucleotides may be used to stabilize a complex.

Some embodiments of the disclosure provide a stabilizing component or biochemical moiety that stabilizes an immune complex, where the sensitivity and/or the specificity of immunoassays is increased. It is understood that the stabilizing component significantly lowers the kinetic dissociation constant (Kd) for an immune complex (e.g., see, FIGs. 1, 2, 3 and 4). The free enthalpy (G) is reduced through lowering the free energy and/or through an entropy driven process due to the additional binding event(s) of the stabilizing component to the immune complex (FIG. 5). Thus, the specific immune complex is stabilized and the chances of detecting this complex are greatly improved.

Two immune complex stabilizing components described in this disclosure are Fc receptors and complementary single-stranded DNA (ssDNA) oligonucleotides (the latter not being part of the present invention). The soluble Fc receptor CD16a was selected as an example. CD16a is expressed on innate immune cells (e.g. macrophages) and binds to immune complexes in order to facilitate downstream immune responses, i.e. opsonization, antibody-dependent cell-mediated cytotoxicity, and complement pathway activation. When complementary ssDNA oligonucleotides hybridize, they form double-stranded DNA with high melting temperatures that are very stable under immunoassay reaction conditions.

The use of stabilizing components may be leveraged in different immunoassays of different architectures (FIGs. 6 and 7). Also, data demonstrate the successful association of CD16a to an immune complex formed using an anti-human IgG monoclonal antibody, subject or patient derived autoimmune thyroid peroxidase antibodies, and a thyroid peroxidase antigen (FIG. 8). The advantage of using stabilizing components is that the limit of detection may be substantially lower on Centaur XP/XPT/CP and ATELLICA IA Analyzer platforms, using existing technology.

### Complexes

One embodiment of the disclosure relates to a complex, comprising:
(a) a target conjugate pair, where the target conjugate pair comprises a target and a conjugate associated with the target; and
(b) a stabilizing component,
   where the stabilizing component binds to the target conjugate pair, where the association constant (Ka) of the complex is greater than the Ka of the complex without the stabilizing component. The complex has a dissociation constant (Kd) less than the Kd of the complex without the stabilizing component, where the difference between the two complexes is the presence of one or more stabilizing components, where the complex comprises one or more stabilizing components, or the complex without a stabilizing complex, i.e., does not have a stabilizing component, or the complex has an association constant (Ka) greater than the Ka of the complex without a stabilizing complex.

One embodiment may be directed to stabilizing components that are either incorporated into a reagent as a formulation component or chemically coupled to critical raw materials associated with the reagent, antibodies, and/or antigens. Another embodiment provides for highly sensitive assays for detecting disease targets or biomarkers.

An embodiment of the disclosure relates to a complex having a Ka that is 2-fold (e.g., 4-fold, 6-fold, 10-fold) or greater than the Ka of the complex without the stabilizing component. Another embodiment may be directed to a Ka of the complex without the stabilizing component that is 2-fold (e.g., 4-fold, 6-fold, 8-fold, or 10-fold) or less than the Ka of the complex. One embodiment may relate to the Kd of the complex without the stabilizing component where the Kd is 2-fold (e.g., 4-fold, 6-fold, 8-fold, or 10-fold) or greater than the Kd of the complex, or the Kd of the complex may be 2-fold (e.g., 4-fold, 6-fold, 8-fold, or 10-fold) or less than the Kd of the complex without the one or more stabilizing components.

In yet another embodiment, the stabilizing components act to stabilize a complex thereby enabling the detection of minute amounts that may be in a sample, increasing the sensitivity and/or specificity of the detection. The stabilizing component significantly lowers the kinetic dissociation constant (Kd) for the complex and increases the association constant (Ka) as compared to the complex without the stabilizing component. Accordingly, the stabilizing components of the disclosure assist by preventing or reducing the unbinding or dissociation of the individual components that form the complex, namely the target, the conjugate associated with the target, and the stabilizing component. In some embodiments, the stabilizing components of the disclosure drive the binding or association of those components of the complex.

Certain embodiments relate to complexes where the stabilizing component may be linked to the target conjugate pair, to the target or target portion of the target conjugate pair, to the conjugate or conjugate portion of the target conjugate pair, or to both the target portion and the conjugate portion of the target conjugate pair. Further embodiments may be directed to the conjugate comprising a target-binding entity linked to a molecule, where the target-binding entity binds to the target through covalent or non-covalent interactions. Any component that associates or binds to the target may be a potential target-binding entity. A person or ordinary skill in the art would understand which targets and target-binding entities would interact. Examples of target-binding entities may include a protein, a nucleic acid, or fragments or portions thereof, an antibody or antibody fragment thereof. Additional examples of a target-binding entity includes: immunoglobulin A (IgA), immunoglobulin D (IgD), immunoglobulin E (IgE), immunoglobulin G (IgG), and immunoglobulin M (IgM); Fab, Fab', F(ab')₂, Fc, Fv, reduced IgG (rIgG), scFv, diabody, triabody, tetrabody, bis-scFv, minibody, Fab₂, Fab3, and heavy chain antibody (hcAb); an antigen (e.g., including recombinant antigens); a receptor; a ligand; DNA, RNA, microRNA, small nucleolar RNA (snoRNA), small nuclear RNA (snRNA), rRNA, tRNA, siRNA, heterogeneous nuclear RNA (hnRNA), shRNA, aptamers, peptide nucleic acids, locked nucleic acids, zDNA, lectin, peptide, dendrimer, membrane protein labeling agent, chemical compound, antibodies, single chain antibodies, monoclonal antibodies, polyclonal antibodies, etc., or a combination thereof.

In one embodiment, the molecule linked to the target-binding entity forms the conjugate of a target conjugate pair. The linked target-binding entity allows for the joining of a molecule, such as one selected from a detectable label, a support, etc., and a target through the target-binding entity.

Another embodiment may provide a detectable label selected from a radioisotope, a chemiluminescent agent, a fluorescent agent, a phosphorescent agent, a chromophore, a fluorophore, or combinations thereof. Examples of a detectable label may include: acridinium, acridinium ester, acridinium sulfonamide, fluorescein, fluorescein isothiocyanate, gold nanoparticles, rhodamine, benzophenoxazine, cyanine 2 (Cy 2), cyanine 3 (Cy 3), cyanine 5 (Cy 5), cyanine 5.5 (Cy 5.5), cyanine 7 (Cy 7), ¹²⁵I, ¹³¹I, ³H, ¹⁴C, ³²P, ³³P, ³⁵S, luminol, luciferin, lucigenin, or derivatives thereof, or any other agents that may be detectable. One of ordinary skill in the art would understand how to label, modify, crosslink, or conjugate detectable labels proteins, nucleic acid sequences,

In yet another embodiment, the support may be used to immobilize the complex, where the complex may be separated from any unbound components, such as target, conjugate, stabilizing component, target conjugate pair, target-binding entity, detectable label. The support allows for a convenient means for removing any unbound components when the complex undergoes washings. Examples of a support or a solid phase include: a bead, a particle, a plate, a tube, or a chip, including crosslinked polymer beads, beaded agarose, planar glass surfaces, nitrocellulose, polystyrene latex, polyvinyl fluoride, diazotized paper, nylon membrane, activated bead, magnetically responsive bead, titanium oxide, silicon oxide, polysaccharide bead, polysaccharide membrane, agarose, glass, polyacrylic acid, polyethylene glycol, polyethylene glycol-polystyrene hybrid, controlled pore glass, glass slide, gold bead, cellulose, and the like. The bead or particle may be used interchangeably, and the magnetically responsive bead may be a paramagnetic bead or a paramagnetic particle.

Another embodiment may provide for a support, e.g., paramagnetic particles (PMP) or magnetized beads that are iron oxide crystals, where the PMP are attracted to a magnetic field. In certain embodiments, the PMP may be coated with an antibody or antigen. Although the use of any support is envisioned, coated PMP provide more reactive surface area than that of coated tubes or beads. An advantage of using the coated PMP is that after binding to the target antigen or antibody, a magnetic field may be applied to the PMP using a magnet, which allows for the PMP to be temporarily immobilized in a container, such as a cuvette. While being held in place, the PMP may be rinsed and washing removes any sample and reagent unbound to the coated PMP complex.

One of ordinary skill in the art would understand the technology and methods for conjugating or linking target-binding entities, either directly or indirectly, to a support, or conjugation of the stabilizing components (e.g., Fc gamma receptors). Embodiments where the target-binding entity is an antibody, a person of skill in the art would have knowledge of the amino acid side chain functionalities or chemical reactive groups useful for linking to a support, one of a binding pair, or a detectable label. Various techniques utilizing, for example, crosslinking chemistry, covalent attachment, functional groups, reactive moieties, amines, sulfhydryls, carboxyls, carbonyls, NHS esters, maleimides, peptide tags, and the like. A support may be attached to a target-binding entity, either directly or indirectly, through covalent or non-covalent interactions.

Yet a further embodiment may provide conjugation of the stabilizing components and their respective target conjugate pair, detectable label, and the like, which may utilize commonly known chemistry. Optimization may be employed by determining which conjugation chemistry, together with molar excess of components, and the addition of spacer arms (including polyethylene glycol) to avoid steric hindrances. For example, frequently used crosslinkers which may be useful in the preparation of various conjugates disclosed here, may include those that target functional groups, such as primary amines, carboxyls, sulfhydryls, and carbonyls.

(not claimed) For example, commonly known and used method for selecting oligonucleotides is Systematic Evolution of Ligands by EXponential enrichment (SELEX). In one embodiment, the aTPOII Class Capture format (Siemens), SELEX may be utilized as follows: first, recombinant thyroid peroxidase antigen (rTPO) may be used as a target and an initial oligonucleotide pool may be incubated with rTPO. Unbound oligonucleotides may then be separated by those bound to rTPO through multiple washings and bound oligonucleotides may then be eluted from the target. Finally, eluted oligonucleotides may be amplified by polymerase chain reaction (PCR). The melting temperature of the eluted oligonucleotides having complementarity may then be determined. Additionally, the methods used to design complementary oligonucleotide primers for PCRs may be employed using melting temperatures that range from 50°C to 80°C and a GC content ranging from 35-65%.

(not claimed) Another embodiment may provide an eluted oligonucleotide with complementarity to the rTPO that is pre-incubated with an acridinium ester labelled rTPO molecule for use in the lite reagent. The complementary ssDNA strand may be introduced to the support by crosslinking the complementary ssDNA to anti-human IgG 8D6. Thus, when a binding event occurs between the support and lite reagent components (capturing TPO autoantibodies (aTPO) from a sample obtained from a subject), the complementary ssDNA strands may anneal due to their proximity, improving stability of the complex.

One disclosure provides for a complex, where the conjugate may be selected from: an antibody linked to a support, an antibody fragment linked to a support, an antibody linked to a support by a binding pair, an antibody linked to a detectable label, or an antigen linked to a detectable label. In another embodiment, the complex provides for two conjugates of the target conjugate pair. A further disclosure provides for a complex having two conjugates that are an antibody fragment linked to a detectable label and an antibody linked to a support by a binding pair. Yet a further disclosure may relate to a complex having two conjugates that are an antigen linked to a detectable label and an antibody linked to a support, where the antibody is directly linked to the support.

One embodiment provides for detectable labels, e.g., acridinium ester, that are covalently bound to a target-binding entity, such as an antibody, where the binding occurs without modifying the ability of the antibody to bind a target antigen.

In one embodiment, a binding pair may link a target-binding entity to a support, where the binding pair may be selected from: biotin/avidin and modifications, derivatives, and analogs thereof; antibody/antigen; ligand/receptor; lectin/polysaccharide; steroid/steroid binding protein; hormone/hormone receptor; immunoglobulin G/Protein A and/or G and/or L; or enzyme/substrate. Another embodiment provides examples of the biotin/avidin and modifications, derivatives, and analogs thereof that may include: biotin, iminobiotin, or desthiobiotin and avidin, streptavidin, flavidin, tamavidin, traptavidin, or commercially available avidin derivatives or modified avidins, such as to NeutrAvidin^{™} (i.e., an avidin derivative that is a deglycosylated and isoelectrically neutral form of avidin; Invitrogen) and CaptAvidin^{™} (i.e., a tyrosine modified avidin; Invitrogen). Additional embodiments provide for binding pairs, where one of a binding pair may be conjugated or coated on to any of the supports disclosed here, these may include serum albumins including bovine serum albumin (BSA), keyhole limpet hemocyanin, immunoglobulin molecules, thyroglobulin, ovalbumin, polysaccharides, polylactic acids, polyglycolic acids, polymeric amino acids, amino acid copolymers, and the like. These binding pairs that may be useful are well known to those skilled in the art, as are the methods of coupling also well known to those of ordinary skill in the art. Other embodiments may be directed to covalently attaching the one of a binding pair to the support using well-known conjugation chemistry. In certain embodiments, the binding pair that may be used to link a target-binding entity to a support is biotin/streptavidin.

Other embodiments provide antibody or antibody fragment thereof of the binding pair, or a target and its corresponding target-binding entity, where the target is an antibody or antibody fragment that binds to its corresponding target-binding entity. Examples of antibody or antibody fragments of the disclosure include: immunoglobulin A (IgA), immunoglobulin D (IgD), immunoglobulin E (IgE), immunoglobulin G (IgG), immunoglobulin M (IgM); Fab, Fab', F(ab')₂, Fc, Fv, reduced IgG (rIgG), scFv, diabody, triabody, tetrabody, bis-scFv, minibody, Fab₂, Fab3, or heavy chain antibody (hcAb).

The stabilizing component may be any component that binds to the target conjugate pair. In the present invention, the stabilizing component is a Fc receptor, which is a receptor for the Fc portion of the IgG. Examples of an FcγR may include a highaffinity receptor, FcyRI (CD64), a low-affinity receptor, FcγRII (CD32), and FcγRIII (CD16), where the FcyRs contain highly conserved extracellular immunoglobulin domains. There are two distinct forms of human CD16 (i.e., CD16a/FcγRIIIa and CD16b/FeyRIIIb). FcγR or CD16 may be found on the surface of natural killer cells, neutrophils, monocytes, mast cells, and macrophages. Another embodiment provides a CD16a or FcγR stabilizing component.

To generally determine a stabilizing component, binding studies may be performed to show that a biochemical agent binds and stabilizes and/or enhances formation of a complex between an analyte and a component that binds the analyte, i.e., analyte binder (e.g. an immune complex or other complexes (e.g., aptamers and analyte, affibodies and analyte, chelator and analyte, etc.)). In some embodiments, secondary monoclonal antibodies such as those that enhance the formation of an antibody-antigen complex may be excluded as acting as stabilizing components including those described in EP0177531, particularly in relation to secondary monoclonal antibodies.

Binding studies need to show that the association constant (Ka) or the dissociation constant (Kd) of the complex between an analyte and the component that binds the analyte is further increased or reduced, respectively, with the addition of the stabilizing component. It may also be shown that the binding velocity of the analyte and the component that binds the analyte is faster or that the dissociation velocity is slower in the presence of the stabilizing component.

For example, surface plasmon resonance (SPR) experiments, e.g. using BIOACORE devices, may be used to show the binding. More specifically, these experiments may be used to determine the association and dissociation rate constants and consequently the association or dissociation constants. Different approaches may also be used, e.g. an antibody may be immobilized and sequentially the analyte and then the stabilizing component may be added to the solution. Binding may be measured through a signal increasing over time and the association rate constants may be determined accordingly. By removing the soluble components from the solution, the dissociation may be measured and consequently, the dissociation rate constants may be determined. Also, the analyte and the stabilizing component may be added simultaneously to determine the combined effect at once. Alternatively, the analyte may be immobilized and the component that binds the analyte and the stabilizing component may be added sequentially or simultaneously. In another embodiment, the stabilizing component may be immobilized and then the binding experiments performed.

A further embodiment may provide the impact of a stabilizing component by demonstrating that the sensitivity of the assay is increased through the addition of the stabilizing component. This increase in sensitivity may be shown through improved analytical sensitivity, limit of blank, limit of detection, or limit of quantitation; whereas, the limit of quantitation may be reduced to show an improvement of the precision of an assay at a low concentration without considering the true measurement, which shows an improvement of the functional sensitivity (Bureau International des Poids et Mesures (BIPM). International Vocabulary of Metrology (VIM) - Basic and General Concepts and Associated Terms. VIM, 3rd edition, JCGM 200:2012; NCCLS. Evaluation of Precision Performance of Quantitative Measurement Methods; Approved Guideline-Second Edition. NCCLS document EP5-A2 (ISBN 1-56238-542-9). NCCLS, 940 West Valley Road, Suite 1400, Wayne, Pennsylvania 19087-1898 USA, 2004.).

Certain embodiments may provide a complex comprising two stabilizing components, which is more stable than a complex with one stabilizing component, and most stable as compared to a complex without any stabilizing component. Specifically, the complex comprising two stabilizing components has a low Kd that increases with the removal of stabilizing components. For example, the Kd values increase from (a) a complex with two stabilizing components < (b) a complex comprising one stabilizing component < (c) a complex without any stabilizing component. Another example may be directed to the Ka values which decrease from (a) a complex with two stabilizing components > (b) a complex comprising one stabilizing component > (c) a complex without any stabilizing component.

Other disclosures may provide for two or more stabilizing components, where some stabilizing components may not have oligonucleotide nucleic acid sequences that are hybridized to those of other stabilizing components, i.e., the stabilizing components may not necessarily be paired up, and they provide a complex with a Kd that is less than the complex without any stabilizing components. For example, a complex comprising a target conjugate pair, where the target conjugate pair comprises a target and a conjugate associated with the target, and three stabilizing components, where two stabilizing components may be hybridized through their oligonucleotide nucleic acid sequences and one stabilizing component comprising an oligonucleotide having a nucleic acid sequence is not paired or hybridized with another oligonucleotide, and the three stabilizing components bind to the target conjugate pair, may have a Ka greater than the Ka of the complex without one stabilizing component, or the Ka of the complex without two stabilizing components, or the Ka of the complex without all three stabilizing components. The Ka of the complex having three stabilizing components may be greater than the Ka of the complex having two stabilizing components, or the Ka of the complex having one stabilizing component, or the Ka of the complex having none or no stabilizing components.

Further embodiments may provide complexes comprising target conjugate pairs, where the target is a biomarker associated with a disease (e.g., disease state, condition, symptoms, and the like). For example, the complexes of the disclosure may comprise two target conjugate pairs, where a first target conjugate pair comprises a thyroid peroxidase antibody (e.g., including autobodies, such as TPO autoantibodies (aTPO)) target and a thyroid peroxidase antigen (e.g., including recombinant antigens, such as recombinant TPO antigen (rTPO)) linked to an acridinium ester and a second target conjugate pair comprises a thyroid peroxidase antibody target and an anti-TPO antigen immunoglobulin G linked to a paramagnetic bead, where the stabilizing component is one or more Fc gamma receptors, where the TPO antigen and the anti-TPO IgG each bind to the TPO antibody target. One FcγR stabilizing component may be conjugated or linked to the TPO antigen linked to acridinium ester detectable label conjugate.

In yet a further embodiment the complexes comprising two target conjugate pairs, where a first target conjugate pair comprises a thyroid peroxidase antibody and a thyroid peroxidase antigen linked to a chip, where the TPO antigen and chip are linked by a biotin/streptavidin binding pair, and a second target conjugate pair comprises an anti-TPO antigen immunoglobulin G linked to an acridinium ester, where the stabilizing component is one or more Fc gamma Receptors, where the TPO antigen and the anti-TPO antigen IgG each bind to the TPO antibody.

Another embodiment may provide a complex, comprising:
(a) two target conjugate pairs, where the target conjugate pair comprises a target and a conjugate associated with the target, where a first target conjugate pair is a thyroid peroxidase antibody (e.g., including autoantibodies, such as TPO autoantibodies (aTPO)) target and a thyroid peroxidase antigen (e.g., including recombinant antigens, such as recombinant TPO antigen (rTPO)) linked to an acridinium ester and a second target conjugate pair is a thyroid peroxidase (TPO) antibody target and an anti-TPO antigen immunoglobulin G linked to a paramagnetic bead, where the TPO antigen and the anti-TPO antigen IgG each bind to the TPO antibody target; and
(b) one or more stabilizing components, where the stabilizing component is a Fc gamma receptor,
   where the one or more stabilizing components bind to the two target conjugate pairs, where the association constant (Ka) of the complex is greater than the Ka of the complex without the one or more stabilizing components.

One embodiment may provide a complex, comprising:
(a) two target conjugate pairs, where the target conjugate pair comprises a target and a conjugate associated with the target, where a first target conjugate pair is a thyroid peroxidase antibody target and a thyroid peroxidase antigen linked to a chip, where the TPO antigen and chip are linked by a biotin/streptavidin binding pair, and a second target conjugate pair is a thyroid peroxidase (TPO) antibody target and an anti-rTPO immunoglobulin G linked to an acridinium ester, where the TPO antigen and the anti-TPO antigen IgG each bind to the TPO antibody target; and
(b) one or more stabilizing components, where the stabilizing component is a Fe gamma receptor,
   where the one or more stabilizing components bind to the two target conjugate pairs, where the association constant (Ka) of the complex is greater than the Ka of the complex without the one or more stabilizing components.

Yet a further embodiment provides a complex, where the conjugate portion of one target conjugate pair is an antibody bound to a support and the conjugate portion of another target conjugate pair is a disease state antigen bound to a detectable label, where the one or more targets may be a circulating disease state antibody that recognizes and binds the disease state antigen, where a stabilizing component binds to the target conjugate pair, and the formed complex has an association constant (Ka) greater than the Ka of the complex without the stabilizing component, or a dissociation constant (Kd) less than the Kd of the complex without the stabilizing component. Another embodiment provides for a circulating disease state antibody target that may be thyroid peroxidase for thyroid disease, where the disease state antigen may be a TPO antigen, where the TPO antigen /TPO antibody form an antigen/antibody pair. In some embodiments, the detectable label, to which the TPO antigen may be linked in a conjugate, may be acridinium ester; the stabilizing component may be an FcR or in particular, an FcyR; the antibody bound to a support may be an anti-TPO antigen IgG bound to a paramagnetic bead.

Various embodiments of the disclosure provide for links, bonds, conjugations, associations, and the like that refer to forces between two or more components that may occur through covalent interactions or non-covalent interactions. Examples of non-covalent interactions include hydrogen bonds, van der Waals interactions, hydrophobic interactions, ion induced dipole, dipole induced dipole, and ionic bonds. While the covalent interactions involve a chemical bond where electron pairs are shared between atoms. Covalent interactions include: hydrogen bonding, σ-bonding, π-bonding, metal-to-metal bonding, agostic interactions, bent bonds, and three-center two-electron bonds. For example, the linkages, bonds, and conjugations between: a target and a target-binding entity, a stabilizing component and a target conjugate pair, a stabilizing component and a target, a stabilizing component and a conjugate, a target-binding entity and a molecule, a target-binding entity and a detectable label, a target-binding entity and a support, a target-binding entity and an oligonucleotide, an antibody and a support, an antibody fragment and a support, an antibody and a binding pair and a support, an antibody and a detectable label, an antigen and a detectable label, and the like, may be covalently or non-covalently bound or linked.

### Methods

The need for highly sensitive detection of targets or biomarkers of disease states, including for example infectious or autoimmune diseases, the methods of the disclosure provide a highly sensitive method using stabilizing components. One disclosure may provide a method, comprising: contacting a sample being tested for the presence of a target and a mixture of a conjugate associated with the target and a stabilizing component, where the target and the conjugate associated with the target form a target conjugate pair, where the stabilizing component is linked to the target conjugate pair to form a complex disclosed here, where the association constant (Ka) of the complex is greater than the Ka of the complex without the stabilizing component. A further embodiment relates to a method of the disclosure, where the dissociation constant (Kd) of the complex without a stabilizing component is 2-fold (e.g., 4-fold, 6-fold, 8-fold, or 10-fold) greater than the Kd of the complex, or the Kd of the complex is 2-fold (e.g., 4-fold, 6-fold, 8-fold, or 10-fold) less than the Kd of the complex without the one or more stabilizing components, or the association constant (Ka) of the complex is 2-fold (e.g., 4-fold, 6-fold, 8-fold, or 10-fold) greater than the Ka of the complex without a stabilizing component, or the Ka of the complex without a stabilizing component is 2-fold (e.g., 4-fold, 6-fold, 8-fold, or 10-fold) less than the Ka of the complex.

One embodiment provides methods where the stabilizing component is linked to the target conjugate pair. In some embodiments, the stabilizing component may be linked to the target or the target portion of the target conjugate pair. Other embodiments provide for a method where the stabilizing component may be linked to the conjugate or conjugate portion of the target conjugate pair. Another embodiment may provide for a method where the stabilizing component is linked to both the target and the conjugate of the target conjugate pair.

Further embodiments may be directed to the methods of the disclosure, where the stabilizing component binds to the target conjugate pair after the formation of the target conjugate pair. Other embodiments may be directed to stabilizing components that bind the conjugate associated with the target prior to the formation of the target conjugate pair.

The conjugate of the disclosure comprises a target-binding entity linked to a molecule, where the target-binding entity binds the target. Examples of the target-binding entity of the disclosed method include: a protein, a nucleic acid, or fragments or portions thereof, an antibody (e.g., immunoglobulin A (IgA), immunoglobulin D (IgD), immunoglobulin E (IgE), immunoglobulin G (IgG), and immunoglobulin M (IgM); antibody fragment is selected from: Fab, Fab', F(ab')₂, Fc, Fv, reduced IgG (rIgG), scFv, diabody, triabody, tetrabody, bis-scFv, minibody, Fab₂, Fab3, and heavy chain antibody (hcAb)); an antigen; a receptor; a ligand; DNA, RNA, microRNA, snoRNA, snRNA, rRNA, tRNA, siRNA, hnRNA, shRNA, aptamers, peptide nucleic acids, locked nucleic acids, zDNA, lectin, peptide, dendrimer, membrane protein labeling agent, chemical compound, antibodies, single chain antibodies, monoclonal antibodies, polyclonal antibodies, etc., or a combination thereof.

Other embodiments of the disclosed methods provide for a conjugate, where the conjugate comprising a target-binding entity linked to a molecule, where the molecule may be selected from: a detectable label, a support, or an oligonucleotide.

Certain embodiments of the disclosure provide methods, where the detectable label of the conjugate may be conjugated to the target-binding entity. Examples of the detectable label include: a radioisotope, a chemiluminescent agent, a fluorescent agent, a phosphorescent agent, a chromophore, a fluorophore, or combinations thereof (e.g., acridinium, acridinium ester, acridinium sulfonamide, fluorescein, fluorescein isothiocyanate, gold nanoparticles, rhodamine, benzoplienoxazine, cyanine 2 (Cy 2), cyanine 3 (Cy 3), cyanine 5 (Cy 5), cyanine 5.5 (Cy 5.5), cyanine 7 (Cy 7), ¹²⁵I, ¹³¹I, ³H, ¹⁴C, ³²P, ³³P, ³⁵S, luminol, luciferin, lucigenin, or derivatives thereof). In one embodiment, the detectable label is an acridinium, an acridinium ester, or an acridinium sulfonamide.

Other embodiments provide methods, where the support may be conjugated either directly or indirectly to the target-binding entity. The support may be selected from: a bead, a particle, a plate, a tube, or a chip (e.g., crosslinked polymer beads, beaded agarose, planar glass surfaces, nitrocellulose, polystyrene latex, polyvinyl fluoride, diazotized paper, nylon membrane, activated bead, magnetically responsive bead, paramagnetic bead, titanium oxide, silicon oxide, polysaccharide bead, polysaccharide membrane, agarose, glass, polyacrylic acid, polyethylene glycol, polyethylene glycol-polystyrene hybrid, controlled pore glass, glass slide, gold bead, cellulose, and the like). In one embodiment, the support may be a bead, microtiter plate, a microtube, or a chip, where the bead may be magnetized, and the magnetic bead may be a paramagnetic bead.

Another embodiment relates to the methods comprising conjugates, where the conjugate may be selected from: an antibody linked to a support, an antibody fragment linked to a support, an antibody linked to a support by a binding pair, an antibody linked to a detectable label, or an antigen linked to a detectable label. One embodiment provides methods comprising two conjugates, where the two conjugates are an antibody fragment linked to a detectable label and an antibody linked to a support by a binding pair. In other embodiments, the conjugates may be an antigen linked to a detectable label and an antibody linked to a support.

Those embodiments of the disclosed methods that utilize a binding pair for linking a target-binding entity to a support may provide binding pairs selected from: biotin/avidin and modifications, derivatives, and analogs thereof; antibody/antigen; ligand/receptor; lectin/polysaccharide; steroid/steroid binding protein; hormone/hormone receptor; immunoglobulin G/Protein A and/or G and/or L; or enzyme/substrate. One embodiment provides for methods where the binding pair links an antibody or fragments thereof, which recognizes a target, to a support, the binding pair is biotin/streptavidin. Other embodiments of the method provide an antibody or antibody fragment thereof selected from: immunoglobulin A (IgA), immunoglobulin D (IgD), immunoglobulin E (IgE), immunoglobulin G (IgG), immunoglobulin M (IgM); Fab, Fab', F(ab')₂, Fc, Fv, reduced IgG (rIgG), scFv, diabody, triabody, tetrabody, bis-scFv, minibody, Fab₂, Fab3, or heavy chain antibody (hcAb).

Further embodiments provide for methods that when contacting a sample and mixture of a conjugate and a stabilizing component, the stabilizing component may bind the target conjugate pair. Alternatively, the stabilizing component may bind the target before the target and conjugate form the target conjugate pair. In other embodiments, the stabilizing component may bind essentially simultaneously to the target and the conjugate as they bind.

In the methods of the invention, the stabilizing component is a Fc receptor (FcR) or a Fc gamma receptor (FcγR), where the FcγR is also known as CD16. Further embodiments may be directed to methods, where the complex comprises two stabilizing components where the stabilizing components may each be a FcR or a FcγR linked to a target-binding entity, or combinations thereof. Another disclosure (not part of the present invention) may relate to methods where the two stabilizing components are two oligonucleotides each linked to the target-binding entity, where the two oligonucleotides comprise complementary nucleic acid sequences. One disclosure may comprise these two oligonucleotides with complementary nucleic acids sequences that are also in sufficient proximity to hybridize. Alternatively, the methods may comprise two oligonucleotides with complementary nucleic acid sequences that are not hybridized, yet still provide some stability to the complex. The target-binding entity linked to the oligonucleotides of the methods disclosed here may include any entity that may bind to the target, including binding pairs and antibodies, where some embodiments utilize IgG antibody target-binding entities that recognize the target of interest. The targets of the disclosure are those of interest or desired when testing samples. These targets may also be considered biomarkers associated with a disease (e.g., disease state, condition, symptoms, etc.)

Certain disclosures may be directed to a molecule comprising a detectable label; an oligonucleotide, where the oligonucleotide comprises a nucleic acid sequence; a support; or a binding pair linking the target-binding protein and the support, where the target-binding protein is indirectly linked to the support via the binding pair. The target-binding protein may, in some embodiments, be selected from an antibody, where the antibody may be an antibody fragment or one of a binding pair, where the binding pair may be selected from biotin/avidin and modifications, derivatives, and analogs thereof, antibody/antigen, ligand/receptor, and enzyme/substrate.

Another disclosure of the method of the disclosure relates to examples of binding pairs which include: the biotin/avidin and modifications, derivatives, and analogs thereof; antibody/antigen; ligand/receptor; lectin/polysaccharide; steroid/steroid binding protein; hormone/hormone receptor; immunoglobulin G/Protein A and/or G and/or L; or enzyme/substrate. Other embodiments provide for examples of biotin/avidin and modifications, derivatives, and analogs thereof which include: biotin, iminobiotin, or desthiobiotin and avidin, streptavidin, flavidin, tamavidin, traptavidin, or commercially available avidin derivatives or modified avidins, such as NeutrAvidin^{™} (i.e., an avidin derivative that is a deglycosylated and isoelectrically neutral form of avidin; Invitrogen) and CaptAvidin^{™} (i.e., a tyrosine modified avidin; Invitrogen).

Another disclosure may relate to the method of the disclosure, where the complex of the disclosure comprises: a target conjugate pair, wherein the target conjugate pair comprises a target and a conjugate associated with the target; and (b) a stabilizing component, where the stabilizing component binds to the target conjugate pair, where the association constant (Ka) of the complex is greater than the Ka of the complex without the stabilizing component, and where the complex has a dissociation constant (Kd) less than the Kd of the complex without the stabilizing component, where the complex comprises a detectable label.

An additional embodiment provides a method further comprising detecting the detectable label, which indicates the presence of the target. In one embodiment, a luminometer may be used to measure the amount of light that the detectable label emits, e.g., by the chemiluminescent reaction.

Yet another embodiment may provide a removing step which removes any components that are not bound to the complex, i.e., unbound to the complex. These unbound components may include targets, target conjugate pairs, stabilizing components, detectable label, and the like. Other embodiments may be directed to a removing step that occurs prior to detecting. Alternatively, the removing step need not be performed. However, including the removing step may reduce background noise and increase the sensitivity of detection.

Another embodiment of the disclosure provides methods for forming the complexes of the disclosure, but also for detecting a target that may be in a sample. The methods of forming a complex of the disclosure may further comprise detecting a detectable label, where the detectable label may be on a conjugate of a target conjugate pair. In other embodiments, the detectable label may be linked to a target-binding entity of a conjugate, such as to an antibody or antibody fragment thereof, where the antibody or antibody fragment thereof bind a target. Other (not part of the present invnetion) may provide methods further comprising detecting a detectable label, where the detectable label may be linked to a stabilizing component, where the stabilizing component is an oligonucleotide linked to a target-binding entity, and the target-binding entity is conjugated to a target. The detectable label, in some embodiments, may be bound to the target-binding entity portion or the oligonucleotide portion of the stabilizing component.

In one embodiment of the disclosure, methods may be provided that comprise: contacting a sample being tested for the presence of a thyroid peroxidase antibody (e.g., including autoantibodies, such as TPO autoantibodies (aTPO)) target and a mixture of a conjugate associated with the target and a Fc gamma Receptor stabilizing component, where the target and the conjugate associated with the target form a target conjugate pair, where the conjugate comprises two conjugates, where a first conjugate is a thyroid peroxidase antigen (e.g., including recombinant antigens, such as recombinant TPO antigen (rTPO)) linked to an acridinium ester detectable label and a second conjugate is an anti-TPO antigen immunoglobulin G linked to a paramagnetic bead, where the TPO antigen and the anti-TPO antigen IgG each bind to the TPO antibody target; wherein the FcγR stabilizing component is linked to the target conjugate pair to form a complex, where the association constant (Ka) of the complex is greater than the Ka of the complex without the stabilizing component. Another embodiment additionally provides a method further comprising detecting the detectable label, e.g., acridinium ester, where the presence of the detectable label indicates the presence of the TPO target. The method in other embodiments may further comprise removing any component that is unbound to the complex or that does not form the complex, where unbound components may include TPO antibody targets, conjugates, FcγR stabilizing components, target conjugate pairs, TPO antigen either alone or linked to acridinium ester detectable label, anti-TPO antigen IgG either alone or linked to a paramagnetic bead, acridinium ester detectable label alone, and/or paramagnetic bead alone. The removing step may occur using reagents that provide for conditions of high, moderate, or low stringency. The conditions typically used in polymerase chain reaction techniques may provide guidance for those of ordinary skill in the art.

Another embodiment of the disclosure provides a method, comprising: contacting a sample being tested for the presence of a thyroid peroxidase antibody target and a mixture of a conjugate associated with the target and a Fc gamma Receptor stabilizing component, where the target and the conjugate associated with the target form a target conjugate pair, where the conjugate comprises two conjugates, where a first conjugate is a TPO antigen bound to a chip, where the TPO antigen and the chip are linked by a biotin/streptavidin binding pair, and a second conjugate is an anti-TPO antigen immunoglobulin G linked to an acridinium ester detectable label, where the FcγR stabilizing component is linked to the target conjugate pair to form a complex, where the association constant (Ka) of the complex is greater than the Ka of the complex without the stabilizing component. A further embodiment additionally provides a method further comprising detecting the detectable label, e.g., acridinium ester, where the presence of the detectable label indicates the presence of the TPO target. The method in other embodiments may further comprise removing any component that is unbound to the complex or that does not form the complex, where unbound components may include TPO antibody targets, conjugates, FcγR stabilizing components, target conjugate pairs, biotin/streptavidin binding pairs or individually, i.e., one of the binding pair, for example, biotin alone or streptavidin alone, TPO antigen either alone or linked to a chip, anti-TPO antigen IgG either alone or linked to an acridinium ester detectable label, acridinium ester detectable label alone, and/or chip alone.

One embodiments of the disclosure (not partof the present invention) provides methods, comprising: contacting a sample being tested for the presence of a thyroid peroxidase antibody target and a mixture of a conjugate associated with the target and a stabilizing component, where the target and the conjugate associated with the target form a target conjugate pair, where the conjugate is a TPO antigen bound to a paramagnetic bead, where the stabilizing component is linked to the target conjugate pair to form a complex, where the stabilizing component is a TPO antigen linked to an oligonucleotide, where the stabilizing component comprises an acridinium ester detectable label, where the association constant (Ka) of the complex is greater than the Ka of the complex without the stabilizing component. A further embodiment additionally provides a method further comprising detecting the detectable label, e.g., acridinium ester, where the presence of the detectable label indicates the presence of the TPO target. The method in other embodiments may further comprise removing any component that is unbound to the complex or that does not form the complex, where unbound components may include TPO antibody targets, conjugates, target conjugate pairs, TPO antigen linked to an oligonucleotide stabilizing components, TPO antigen either alone or linked to a paramagnetic bead, TPO antigen either alone or linked to an acridinium ester detectable label, acridinium ester detectable label alone, and/or paramagnetic bead alone. The removing step may occur using reagents that provide for conditions of high, moderate, or low stringency.

Certain embodiments of the disclosure may provide methods further comprising removing any unbound components of the complex, where the unbound components may include a target, a stabilizing component, a conjugate, a conjugate pair, a detectable label, a binding pair, one of a binding pair, etc. The removal step reduces any background noise and allows for increased sensitivity, where components provided in excess may remain in the mixture. The removing step generally would involve washing away any unbound components, where the washing comprises multiple washes, using various solutions under conditions that either enable specific binding (e.g., high stringency conditions) or less specific or non-specific binding (e.g., moderate or low stringency conditions). The washing conditions may include those sufficient to remove any materials unbound or not bound to the support, yet not affecting the binding of the materials to the support or the linkages of materials to each other. Generally, the washing conditions and their stringency may be similar to those used for hybridization by nucleic acid compositions. The stringency may include low, moderate, or high levels of stringency depending on the factors such as the nature of the molecule, protein, or nucleic acid sequence, the concentration of salts, the presence or absence of other reaction components (e.g., formamide, dextran sulfate, polyethylene glycol) and reaction temperature (e.g., within a range of from about 5°C below the melting temperature of the molecule or reagent binding to the support to about 20°C to 25°C below the melting temperature). One or more factors may be modified to generate conditions, i.e., low, moderate, or high stringency, that are different from but equivalent to the aforementioned conditions. Conditions of high stringency or optimal binding refer to conditions that permit specific binding of those target-binding proteins, agents, and the like, and a support. These specific binding conditions may occur at a high pH (e.g., pH greater than neutral), an incubation period of several hours, a temperature ranging from 4°C to 37°C, where a lower temperature for a longer period of incubation time may more optimal for specific binding, and/or the like. Alternatively, low stringency conditions or less optimal binding conditions allow for non-specific binding between target-binding proteins, agents, and the like, and a support, may occur at a lower pH (e.g., pH less than neutral), an incubation period of several minutes, and a higher temperature range (e.g., 20°C to 25°C or greater) for a shorter period of incubation time for non-specific binding to a support.

In some embodiments, following the removal of unbound materials and any optional washes, the method may further comprise detecting the one or more detectable labels, thereby identifying the presence of the one or more targets, where the complexes bound to a support may be detected using any known detection method to detect the detectable label of the complex.

Another embodiment additionally provides a method further comprising a detecting the detectable label, where the presence of the detectable label indicates the presence of the target. Some embodiments of the method provide for, prior to detecting, removing any target, stabilizing component, conjugate, or target conjugate pair unbound from the complex. Alternatively, a removing step may not need to be performed before the detecting step or at all.

In one embodiment of the disclosed methods, binding pairs for binding a target-binding entity to a support may include: serum albumins including bovine serum albumin (BSA), keyhole limpet hemocyanin, immunoglobulin molecules, thyroglobulin, ovalbumin, and the like. Other agents that may be used to bind the target-binding entity to the support include polysaccharides, polylactic acids, polyglycolic acids, polymeric amino acids, amino acid copolymers, etc. These binding pairs and reagents are well known to those skilled in the art, as are the methods of binding pairs are well known to those of ordinary skill in the art. Other embodiments may be directed to covalently attaching the target-binding protein to the support using well-known conjugation chemistry. The supports of the disclosure may be particularly useful in the separation of the complex and any complexes unbound or not bound to the support. In one embodiment, magnetic beads, such as for example paramagnetic beads, may be used to separate the complex that is immobilized to a support from any unbound molecules, including unbound target-binding conjugates, unbound stabilizing components, unbound targets, and the like, where the complex immobilized to the support may be detectably labeled thereby enabling the detection of the complex comprising the target.

Other embodiments may provide any conventional immunoassay or detection method further modified to detect and quantitate the desired target indicative of a disease, disease state, or condition, as well as, if needed, one or more control samples or references in accordance with the disclosure. The modifications necessary would be to include contacting stabilizing components that assist in reducing the dissociation of the complex as compared to the complex without the stabilizing components. A person of ordinary skill in the art would have the knowledge and technical background for modifying and carrying out the methods of the disclosure. For example, a reference may be a positive control and/or a negative control for the target being tested, a known quantity of the target so that the amount tested in a sample may be compared for quantification, and the like, or combinations thereof. Assays well known in the art, such as ELISAs, competitive EIAs, RIAs, sandwich assays, including those having dual antibodies, among other assays commonly used in the diagnostic industry, may be modified by the addition of one or more stabilizing components in order to drive the binding constant to formation of a complex with the one or more stabilizing components, and avoid or reduce the dissociation of the components that form the complex. Certain embodiments may utilize immunoassays where the detectable label may be conjugated to a target-binding protein by a fluorescent tag, enzyme, or chemiluminescent tag. Automated immunoassays are also envisioned.

In another embodiment, known methods of detecting may be modified by a person of ordinary skill in the art according to the disclosure. For example, one or more wells of a microtiter plate, or the surface of a bead, a chip, or a tube may be coated with one of a binding pair, such as, streptavidin, to form a support that may be recognized by a conjugate. Various entities are added to the microtiter plate, chip, or container holding beads, including: the conjugate comprising the other of the binding pair conjugated to a target-binding entity such as an antibody, where the other of the binding pair is, in this example, biotin which binds to the immobilized streptavidin, where the antibody is immunoglobulin that recognizes the desired target; another conjugate comprising a target-binding entity, such as an antibody fragment (e.g., F(ab')₂), conjugated to a detectable label (e.g., acridinium ester) molecule, where the antibody fragment recognizes the desired target; a sample obtained from a subject for testing for the presence of one or more desired targets; and one or more stabilizing components (e.g., FcγR). The various entities may be added simultaneously or sequentially and may be incubated at varying times. For convenience, all of the entities for testing may be added simultaneously or essentially simultaneously, where no separate incubations are necessary before the addition of the various entities. If a target of interest is present in the sample, a complex immobilized to the support, such as the microtiter plate, forms, where the complex comprises: (1) a target bound to the support via an antibody linked by a biotin/streptavidin binding pair; (2) the target is also bound to a detectable label via an antibody fragment, where the antibody fragment recognizes the target; and (3) one or more stabilizing components, which may recognize the antibody and antibody fragment of the target conjugate pairs, and the detection of the detectable label indicates the presence of the desired target.

In one embodiment and without being bound by theory, when the target conjugate pair forms, a conformational change may occur, thereby allowing the one or more stabilizing components to bind to the target conjugate pair. Prior to target conjugate pair formation, a conformational change likely does not occur, and the stabilizing component cannot bind to the target conjugate pair. Other embodiments may provide a conjugate that is linked to a stabilizing component before binding a target, where the conjugate may be, for example, a target-binding entity linked to a detectable label. Certain embodiments may provide for a stabilizing component crosslinked to the conjugate comprising, for example, a target-binding entity linked to a detectable label.

Another embodiment may be directed to the use of a support of magnetized beads, for example, paramagnetic beads. The magnetized beads may be conjugated directly to an antibody that recognizes the target of interest (or target-binding entity), or indirectly via a binding pair. A person of ordinary skill in the art would understand that a magnet may be used to temporarily immobilize the magnetized beads comprising the complex of the disclosure in, for example, a tube or cuvette during a removal step, where any components not bound to the magnetized beads are washed or rinsed away until the magnet immobilizing the magnetized beads is released after washing. The removal step would reduce any background noise that may result during a subsequent detecting step. If using, for example, a chemiluminescent agent or chromophore as a detectable label and the detectable label is linked to the complex, detection using a luminometer would identify and detect the complex, thereby indicating the presence of the desired target in the sample,

Other embodiments provide methods that may be adapted for automation. The above-outlined methods may be embodied in automated formats. Particular embodiments provide automated immunoassay systems to which the methods of this disclosure may be adapted, such as those using chemiluminescence and magnetic separation.

One disclosure (not part of the present invention) may provide use of a stabilizing component in the detection of a target of interest. Another disclosure may be directed to the use of a stabilizing component in the detection of a target of interest, where a complex comprising (a) a target conjugate pair, where the target conjugate pair comprises a target and a conjugate associated with the target; and (b) a stabilizing component, where the stabilizing component binds to the target conjugate pair, and the complex is detectably labeled, where the association constant (Ka) of the complex is greater than the Ka of the complex without the stabilizing component.

### Targets

Any known disease state target or biomarker may be tested for in a sample, such as from a subject or patient potentially suffering from a disease associated with the disease state target or biomarker. The target may be an antibody, such as autoantibodies. The sample may also be used for characterizing a particular phenotype. Various embodiments of the disclosure relate to biomarkers or disease-associated targets in a sample that may be analyzed in order to provide a diagnosis and/or prognosis of a disease, disease state, or condition associated with the particular biomarker or target. These targets may include circulating proteins or nucleic acids.

In some embodiments, a sample obtained from a subject may be any bodily fluid or any sample in fluid form. Examples of samples include: peripheral blood, sera, plasma, ascites, urine, cerebrospinal fluid (CSF), sputum, saliva, bone marrow, synovial fluid, aqueous humor, amniotic fluid, cerumen, breast milk, broncheoalveolar lavage fluid, semen, prostatic fluid, cowper's fluid or pre-ejaculatory fluid, female ejaculate, sweat, fecal matter, hair, tears, cyst fluid, pleural and peritoneal fluid, pericardial fluid, lymph, chyme, chyle, bile, interstitial fluid, menses, pus, sebum, vomit, vaginal secretions, mucosal secretion, stool water, pancreatic juice, lavage fluids from sinus cavities, bronchopulmonary aspirates, blastocyl cavity fluid, or umbilical cord blood.

Another embodiment may be directed to examples of diseases that may be tested for biomarkers or disease state targets include: thyroid disease, including hyperthyroidism, hypothyroidism, autoimmune disorders, such as Hashimoto's disease or Graves' disease (e.g., thyroid peroxidase (TPO), Tg, Free T3, Free T4, Total T3, Total T4, TsH3-ultra, TSH, T uptake), hepatitis (e.g., HBe, HBs 2, HAV IgM, HAV Total, HBc IgM, HBc Total, HBeAg, HBsAg confirmatory, HBsAgII, HBsAgII Quant, HCV), HIV (e.g., HIV 1/O/2 Enhanced (EHIV), HIV Combo (CHIV)), cancers (e.g., AFP, BR 27.29, CA 125II, CA 15-3, CA 19-9, Calcitonin, CEA, Complexed PSA, Free PSA, PSA, Serum HER-2/neu), prostate cancer (e.g., prostate-specific antigen (PSA)), ovarian cancer (e.g., CA125), squamous cell carcinoma of the head and neck (SCCHN) (e.g., macrophage inflammatory protein 1b (MIP 1b), interleukin 13 (IL13), metalloproteinase 3 (MMP3), epidermal growth factor (EGF) vascular cell adhesion molecule (VCAM), squamous cell carcinoma antigen (SCC-Ag) and neurite growth-promoting factor 2 (Midkine)), and squamous cell carcinoma of the tongue (SCCT) (e.g., NT-3, TNFB, CD5, uPA, IL-1ra, Flt3L, DNER, CXCL1, CD6, CD40, CDH3, PECAM-1, TIE2, FasL, FR-alpha, CD69, CD244, TWEAK, IL-12B, EGFR, NTRK3, ErbB4/HER4, IL-12), diabetes (e.g., C-Peptide, insulin), Kidney Risk Inflammatory Signature (KRIS) which may indicate the risk of progression of diabetic kidney disease to end stage renal disease (e.g., TNF-RSF1A, TNF-RSF1B, TNF-RSF21, TNF-RSF19, TNF-RSF27, TNF-RSF19L, IL-15RA, IL-17F, CD55, CD300C, TNF-SF15, CCL14, CCL15, CSF1, HAVCR2, IL-1R1, IL-18R1), cardiovascular disease, such as, Atherosclerotic Cardiovascular Disease (ASCVD), heart failure, etc. (e.g., growth differentiation factor 15 (GDF15), tissue inhibitor of metalloproteinase-1 (TIMP1), beta-2-microglobulin (B2M), NT-proBNP, adrenomedullin (ADM), C-type lectin domain family 3 member B (CLEC3B), insulin-like growth factor 1 (IGF1), butyrylcholinesterase (BCHE), paraoxonase 1 (PON1), insulin-like growth factor binding protein 1 (IGFBP1), IGFBP2, IGFBP3, CNTN1, kallikrein B1 (KLKB1), and peripheral myelin protein 2 (PMP2), REG1A, Cystatin-C, d_troponin, AGP1, sICAM1, CRP, CD5L, CD14, MPO, UCMGP, EFEMP1, GRN, Adipsin, Resistin, A1M, d_IL6, FGF23, MMP8, MMP9, SAA1, BCHE, PON1, MCP1, sGP130, Ceruloplasmin, COL18A1, NRCAM, CDH13, LDLR, d FLT3, FBN, d_CSF2RB, β-adrenergic receptors (βARs) (norepinephrine endogenous ligand), Angiotensin II type 1 receptors (AT1Rs) (AngII endogenous ligand), Alzheimer's disease and/or mild cognitive impairment (e.g., ONECUT2, SIRT1, BCL2, PSEN1), tumor angiogenesis (e.g., delta-like 4 (DLL4)), etc. Some of these biomarkers or targets may be increased or decreased in a subject suffering from a disease or condition associated with the marker or target being tested. As these targets are known in the art, a person of ordinary skill in the art would be sufficiently knowledgeable to interpret the results of the detection methods of the disclosure and evaluate or monitor the progression of the disease state and the like. However, the methods of detection described here are directed to the improved sensitivity and accuracy of detection of targets or biomarkers that may not be easily detected.

### Kits

In other embodiments of the disclosure, kits comprising the various components for detecting the potential presence of disease targets or biomarkers and quantifying them are also provided. The present invention provides for a kit comprising: a conjugate known to associate with a particular target and a stabilizing component, as defined in the claims, and optionally, reagents, including those for attaching or detecting detectable labels, washing or rinsing components of the detection method, and diluents; a support; positive and/or negative controls or references; containers; and any operating instructions and/or equipment necessary for executing the methods disclosed here. The kits of the disclosure may also contain conjugates comprising a target-binding entity linked to a molecule, where the target-binding entity binds to a desired target, where the conjugate may include: an antibody linked to a support, an antibody fragment linked to a support, an antibody linked to a support by a binding pair, an antibody linked to a detectable label, or an antigen linked to a detectable label, where the antigen includes recombinant antigens. Binding pairs for linking components to a support, to a detectable label, or to a target are also provided in some of the kit embodiments. In one disclosure, a kit comprises a mixture of a conjugate associated with the desired target and a stabilizing component, a conjugate comprising a target-binding entity linked to a detectable label, a conjugate comprising a target-binding entity linked to a support, where the target-binding entity is configured to bind the desired target, where the stabilizing component may be a protein or receptor that may bind to a target conjugate pair.

A kit of the disclosure provides any of the components utilized in the methods of the disclosure except for the desired target, which would potentially be in a sample for testing. However, a person of ordinary skill in the art would understand how to select the components for detecting a particular and desired target. For example, if thyroid peroxidase (TPO) antibody (e.g., including autoantibodies, such as TPO autoantibodies (aTPO)) is the desired target for testing the presence in a sample, a kit comprising a Fc gamma receptor stabilizing component, a TPO antigen (e.g., including recombinant antigens, such as recombinant TPO antigen (rTPO)) linked to a detectable label, such as for example, an acridinium ester, an anti-TPO antigen IgG linked to a support, such as for example, a paramagnetic bead, where the TPO antigen and the anti-TPO antigen IgG may bind to the desired TPO antibody target.

Another embodiment provides for a kit comprising Fc gamma receptor as stabilizing component, a TPO antigen linked to a detectable label, such as for example, an acridinium ester, an anti-TPO antigen IgG linked to a support, such as for example, a chip, where the TPO antigen and the chip are linked by a biotin/streptavidin binding pair, the chip may have streptavidin attached and the TPO antigen may be linked to biotin, where the TPO antigen and the anti-TPO antigen IgG may bind to the desired TPO antibody target

A further embodiment provides for a kit comprising a TPO antigen bound to a support, e.g., paramagnetic bead, where the stabilizing component is a TPO antigen linked to an oligonucleotide, where the stabilizing component may further be conjugated to a detectable label, e.g., acridinium ester, where the TPO antigen is known to bind to the desired TPO antibody.

Additionally, embodiments of the disclosure may also be directed to an initial investment in equipment and/or software for use with the kits of the disclosure. Further elements of the kit may be incorporated, but as they are typically available in a facility that would be performing the disclosed detection and quantification assays, they are not required or may be sold separately. These elements may include containers, cuvettes, tips, pipettes, tips, disposable gloves, calibrator reagents, quality control materials, reagents for creating a concentration curve, and the like.

### EXAMPLES

The following examples illustrate specific aspects of the instant description. The example merely provides specific understanding and practice of the embodiments and its various aspects.

### Example 1: Sensorgram demonstrating stability of complexes

For studying protein-protein interactions and for determining their equilibrium and kinetic parameters, an assay was performed, and the results of which are shown in the sensorgram of FIG. 8. Complexes of the disclosure demonstrated an increase in equilibrium association constant (Ka) of a complex comprising stabilizing components as compared to the Ka of the complex without stabilizing components or a decrease in dissociation constant (Kd) of complex without stabilizing components as compared to the Kd of the complex with stabilizing components. The association profiles shown in the sensorgram demonstrate a sustainable immune complex.

A sensor chip (BioCore) was pre-loaded with streptavidin and a biotin conjugated to recombinant thyroid peroxidase (rTPO) was added to the chip. A thyroid peroxidase autoantibody (aTPO) positive spiker was then injected onto the chip, followed by extensive washing using a reagent buffer to eliminate non-specific bindings. After removing any non-specific associations, anti-human immunoglobulin G (anti-hIgG) monoclonal antibody clone 4H5 was first injected and then followed by injection of CD16a at a concentration of 20 µg/mL. The injection and washing was performed using the eHIV lite reagent buffer, which contains an TPO antibody linked to a detectable label, e.g., acridinium ester (Siemens; ADVIA Centaur XP Immunoassay System). The chemiluminescent reaction was initiated by the addition of acid and base. The emission of light was measured in relative light units (RLUs) by a luminometer. Generally, the quantitation of light resulted from the oxidation of acridinium ester.

### Example 2: Capture Assay Architecture

A capture assay method (e.g., aTPOII Class Capture assay; Siemens) utilized the following conjugates: for lite reagent and support, respectively: recombinant thyroid peroxidase antigen (rTPO) conjugated to an acridinium ester (i.e., NSP-DMAE-Z) at a molar excess of 20x using N-hydroxysuccinimide (NHS) chemistry and anti-human IgG, 8D6, directly coupled to paramagnetic particles. A typical reaction using an aTPOII reagent pack run on a Centaur XP instrument (Siemens) would proceed as shown in FIG. 9A. The reaction would proceed as follows: (1) 30µL of subject or patient sample potentially containing TPO autoantibodies (aTPO) was incubated in an empty cuvette for 4.75 minutes, (2) 200µL of support reagent (SP) (e.g., target-binding entity (e.g., anti-rTPO IgG) conjugated to support (e.g., paramagnetic particle (PMP)) was added, (3) subject sample and support reagent were incubated for 2.75 minutes, (4) 100µL of the lite reagent (LR) (e.g., rTPO conjugated to acridinium ester (AE) detectable label) was added, (5) subject sample, lite reagent, and support was incubated for 6.5 minutes, and (5) the pellet was washed to separate unbound components; acid and base were added to activate the chemiluminescent reaction; and then the emitted light from the reaction was read in a luminometer. The introduction of FcγR/CD16a into this immune reaction occurred as shown in FIG. 9B, with the stabilizing component, CD16a, conjugated to rTPO-AE.

## Claims

1. An immune complex, comprising:
(a) a target conjugate pair, wherein the target conjugate pair comprises a target and a conjugate associated with the target; and
(b) a stabilizing component,
wherein the stabilizing component binds to the target conjugate pair, wherein the association constant (Ka) of the complex is greater than the Ka of the complex without the stabilizing component, wherein the target is an antigen,
wherein the conjugate comprises a target-binding entity that is an antibody or antibody fragment that recognizes the antigen target and binds to the target, and
wherein the stabilizing component is a Fc receptor linked to the target binding entity.

2. The complex of claim 1, wherein the target-binding entity is linked to a molecule and the molecule is selected from: a detectable label, a support, or an oligonucleotide, wherein the oligonucleotide comprises a nucleic acid sequence of 20 to 100 nucleotides.

3. The complex of any one of claims 1-2, wherein the conjugate comprises a binding pair selected from: biotin/avidin and modifications, derivatives, and analogs thereof; antibody/antigen; ligand/receptor; lectin/polysaccharide; steroid/steroid binding protein; hormone/hormone receptor; immunoglobulin G/Protein A and/or G and/or L; or enzyme/substrate, wherein the binding pair is preferably biotin/streptavidin.

4. The complex of any one of claims 1-3, wherein the antibody or antibody fragment thereof is selected from: immunoglobulin A (IgA), immunoglobulin D (IgD), immunoglobulin E (IgE), immunoglobulin G (IgG), immunoglobulin M (IgM); Fab, Fab', F(ab')₂, Fc, Fv, reduced IgG (rlgG), scFv, diabody, triabody, tetrabody, bis-scFv, minibody, Fab₂, Fab3, or heavy chain antibody (hcAb).

5. The complex of any one of claims 1-4, wherein the Fc receptor is a Fc gamma receptor (FcγR).

6. The complex of any one of claims 1-5, wherein the complex comprises two target conjugate pairs, wherein a first target conjugate pair comprises a thyroid peroxidase (TPO) antibody and a thyroid peroxidase antigen linked to an acridinium ester and a second target conjugate pair comprises a thyroid peroxidase antibody and an immunoglobulin G linked to a paramagnetic bead, wherein the stabilizing component is one or more Fc gamma receptors (FcyRs), wherein the TPO antigen and the IgG each bind to the TPO antibody.

7. The complex of any one of claims 1-6, wherein the complex comprises two target conjugate pairs, wherein a first target conjugate pair comprises a thyroid peroxidase antibody and a thyroid peroxidase antigen linked to a chip, wherein the TPO antigen and chip are linked by a biotin/streptavidin binding pair, and a second target conjugate pair comprises an immunoglobulin G linked to an acridinium ester, wherein the stabilizing component is one or more Fc gamma Receptors (FcyRs), wherein the TPO antigen and the IgG each bind to the TPO antibody.

8. A method, comprising: contacting a sample being tested for the presence of a target and a mixture of a conjugate associated with the target and a stabilizing component, wherein the target and the conjugate associated with the target form a target conjugate pair, wherein the stabilizing component is linked to the target conjugate pair to form a complex of any one of claims 1-7, wherein the association constant (Ka) of the complex is greater than the Ka of the complex without the stabilizing component.

9. The method of claim 8, further comprising: detecting the acridinium ester, thereby indicating the presence of the target.

10. The method of claim 8 or 9, further comprising removing any components unbound from the complex, wherein the removing occurs preferably prior to the detecting.

11. A kit comprising a conjugate associated with a target and a stabilizing component as defined according to any one of claims 1-7.

## Patentansprüche

1. Immunkomplex, umfassend:
(a) ein Zielkonjugatpaar, wobei das Zielkonjugatpaar ein Ziel und ein mit dem Ziel verknüpftes Konjugat umfasst; und
(b) eine Stabilisierungskomponente,
wobei die Stabilisierungskomponente an das Zielkonjugatpaar bindet, wobei die Assoziationskonstante (Ka) des Komplexes größer ist als die Ka des Komplexes ohne die Stabilisierungskomponente, wobei das Ziel ein Antigen ist,
wobei das Konjugat eine Zielbindungsentität ist, die ein Antikörper oder ein Antikörperfragment ist, der/das das Antigenziel erkennt und an das Ziel bindet, und
wobei die Stabiliserungskomponente ein Fc-Rezeptor ist, der mit der Zielbindungsentität verknüpft ist.

2. Komplex nach Anspruch 1, wobei die Zielbindungsentität mit einem Molekül verknüpft ist und das Molekül ausgewählt ist aus: einem nachweisbaren Marker, einem Träger und einem Oligonukleotid, wobei das Oligonukleotid eine Nukleinsäuresequenz von 20 bis 100 Nukleotiden umfasst.

3. Komplex nach einem der Ansprüche 1-2, wobei das Konjugat ein Bindungspaar umfasst, ausgewählt aus: Biotin/Avidin und Modifizierungen, Derivaten und Analoga davon; Antikörper/Antigen; Ligand/Rezeptor; Lektin/Polysaccharid; Steroid/Steroid bindendes Protein; Hormon/Hormonrezeptor; Immunglobulin G/Protein A und/oder G und/oder L; oder Enzym/Substrat, wobei das Bindungspaar vorzugsweise Biotin/Streptavidin ist.

4. Komplex nach einem der Ansprüche 1-3, wobei der Antikörper oder das Antikörperfragment davon ausgewählt ist aus: Immunglobulin A (IgA), Immunglobulin D (IgD), Immunglobulin E (IgE), Immunglobulin G (IgG), Immunglobulin M (IgM); Fab, Fab', F(ab')₂, Fc, Fv, reduziertem IgG (rIgG), scFv, Diabody, Triabody, Tetrabody, bis-scFv, Minibody, Fab₂, Fab3, oder Antikörper der schweren Kette (hcAb).

5. Komplex nach einem der Ansprüche 1-4, wobei der Fc-Rezeptor ein Fc-Gamma-Rezeptor (FcγR) ist.

6. Komplex nach einem der Ansprüche 1-5, wobei der Komplex zwei Zielkonjugatpaare umfasst, wobei ein erstes Zielkonjugatpaar einen Thyroidperoxidase(TPO)-Antikörper und ein mit einem Acridiniumester verknüpftes Thyroidperoxidase-Antigen umfasst, und wobei ein zweites Zielkonjugatpaar einen Thyroidperoxidase-Antikörper und ein mit einem paramagnetischen Bead verknüpftes Immunglobulin G umfasst, wobei die Stabilisierungskomponente ein oder mehrere Fc-Gamma-Rezeptoren (FcγRs) ist, wobei das TPO-Antigen und das IgG jeweils an den TPO-Antikörper binden.

7. Komplex nach einem der Ansprüche 1-6, wobei der Komplex zwei Zielkonjugatpaare umfasst, wobei ein erstes Zielkonjugatpaar einen Thyroidperoxidase-Antikörper und ein mit einem Chip verknüpftes Thyroidperoxidase-Antigen umfasst, wobei das TPO-Antigen und der Chip über ein Biotin/Streptavidin-Bindungspaar verknüpft sind, und wobei ein zweites Zielkonjugatpaar ein mit einem Acridiniumester verknüpftes Immunglobulin G umfasst, wobei die Stabilisierungskomponente ein oder mehrere Fc-Gamma-Rezeptoren (FcγRs) ist, wobei das TPO-Antigen und das IgG jeweils an den TPO-Antikörper binden.

8. Verfahren, umfassend: Inberührungbringen einer auf das Vorliegen eines Ziels getesteten Probe mit einer Mischung aus einem mit dem Ziel assoziierten Konjugat und einer Stabilisierungskomponente, wobei das Ziel und das mit dem Ziel assoziierte Konjugat ein Zielkonjugatpaar ausbilden, wobei die Stabilisierungskomponente mit dem Zielkonjugatpaar verknüpft ist, um einen Komplex nach einem der Ansprüche 1-7 auszubilden, wobei die Assoziationskonstante (Ka) des Komplexes größer ist als die Ka des Komplexes ohne die Stabilisierungskomponente.

9. Verfahren nach Anspruch 8, ferner umfassend: Nachweisen des Acridiniumesters und dadurch Nachweisen des Vorliegens des Ziels.

10. Verfahren nach Anspruch 8 oder 9, ferner umfassend das Entfernen beliebiger nicht an den Komplex gebundener Komponenten, wobei das Entfernen vorzugsweise vor dem Nachweisen stattfindet.

11. Kit, umfassend ein Konjugat, das mit einem Ziel verknüpft ist, und eine Stabilisierungskomponente nach einem der Ansprüche 1-7.

## Revendications

1. Complexe immunitaire comprenant :
a) une paire de conjugués cibles, dans lequel la paire de conjugués cibles comprend une cible et un conjugué associé à la cible ; et
b) un composant stabilisateur,
dans lequel le composant stabilisateur se lie à la paire de conjugués cibles, dans lequel la constante d'association (Ka) du complexe est supérieure à la Ka du complexe sans le composant stabilisateur, dans lequel la cible est un antigène,
dans lequel le conjugué comprend une entité de liaison de cible qui est un anticorps ou un fragment d'anticorps qui reconnaît la cible d'antigène et se lie à la cible, et
dans lequel le composant stabilisateur est un récepteur Fc lié à l'entité de liaison de cible.

2. Complexe de la revendication 1, dans lequel l'entité de liaison de cible est liée à une molécule et la molécule est choisie parmi les éléments suivants : une étiquette détectable, un support ou un oligonucléotide, dans lequel l'oligonucléotide comprend une séquence d'acide nucléique de 20 à 100 nucléotides.

3. Complexe de l'une quelconque des revendications 1 à 2, dans lequel le conjugué comprend une paire de liaison choisie parmi les éléments suivants : la biotine/l'avidine et ses modifications, ses dérivés et ses analogues ; l'anticorps/l'antigène ; le ligand/le récepteur ; la lectinelle polysaccharide ; le stéroïde/la protéine de liaison aux stéroïdes ; l'hormonelle récepteur hormonal ; l'immunoglobuline Glla protéine A et/ou G et/ou L ; ou l'enzyme/le substrat, dans lequel la paire de liaisons est de préférence la biotine/la streptavidine.

4. Complexe de l'une quelconque des revendications 1 à 3, dans lequel l'anticorps ou le fragment d'anticorps est choisi parmi les éléments suivants : l'immunoglobuline A (IgA), l'immunoglobuline D (IgD), l'immunoglobuline E (IgE), l'immunoglobuline G (IgG), l'immunoglobuline M (IgM) ; Fab, Fab', F(ab')₂, Fc, Fv, IgG réduite (rlgG), scFv, le dianticorps, le trianticorps, le tétraanticorps, bis-scFv, minicorps, Fab₂, Fab₃, ou l'anticorps à chaîne lourde (hcAb).

5. Complexe de l'une quelconque des revendications 1 à 4, dans lequel le récepteur Fc est un récepteur gamma Fc (FcγR).

6. Complexe de l'une quelconque des revendications 1 à 5, dans lequel le complexe comprend deux paires de conjugués cibles, dans lequel une première paire de conjugués cibles comprend un anticorps de thyroïde peroxydase (TPO) et un antigène de thyroïde peroxydase lié à un ester d'acridinium et une deuxième paire de conjugués cibles comprend un anticorps de thyroïde peroxydase et une immunoglobuline G liée à une bille paramagnétique, dans lequel le composant stabilisateur est un ou plusieurs récepteurs gamma Fc (FcyRs), dans lequel l'antigène TPO et l'IgG se lient chacun à l'anticorps TPO.

7. Complexe de l'une quelconque des revendications 1 à 6, dans lequel le complexe comprend deux paires de conjugués cibles, dans lequel une première paire de conjugués cibles comprend un anticorps de thyroïde peroxydase et un antigène de thyroïde peroxydase lié à une puce, dans lequel l'antigène TPO et la puce sont liés par une paire de liaison de la biotine/la streptavidine, et une deuxième paire de conjugués cibles comprenant une immunoglobuline G liée à un ester d'acridinium, dans lequel le composant stabilisateur est un ou plusieurs Récepteurs gamma Fc (FcγRs), dans lequel l'antigène TPO et l'IgG se lient chacun à l'anticorps TPO.

8. Procédé comprenant : la mise en contact d'un échantillon testé pour la présence d'une cible et d'un mélange d'un conjugué associé à la cible et d'un composant stabilisateur, dans lequel la cible et le conjugué associé à la cible forment une paire de conjugués cibles, dans lequel le composant stabilisateur est lié à la paire de conjugués cibles pour former un complexe de l'une quelconque des revendications 1 à 7, dans lequel la constante d'association (Ka) du complexe est supérieure à la Ka du complexe sans le composant stabilisateur.

9. Procédé de la revendication 8, comprenant en outre : la détection de l'ester d'acridinium, indiquant ainsi la présence de la cible.

10. Procédé de la revendication 8 ou 9, comprenant en outre l'élimination de tout composant non lié au complexe, dans lequel l'élimination se produit de préférence avant la détection.

11. Kit comprenant un conjugué associé à une cible et un composant stabilisant tel que défini selon l'une quelconque des revendications 1 à 7.
